(19) Europäisches Patentamt
European Patent Office
Office européen des brevets

(11) Numéro de publication : **0 540 629 B1**

# (12) FASCICULE DE BREVET EUROPEEN

(45) Date de publication du fascicule du brevet : **04.05.94 Bulletin 94/18**

(51) Int. Cl.$^5$ : **A61K 7/06**, A61K 31/505, C07D 239/48

(21) Numéro de dépôt : **91913941.0**

(22) Date de dépôt : **19.07.91**

(86) Numéro de dépôt international : **PCT/FR91/00596**

(87) Numéro de publication internationale : **WO 92/01437 06.02.92 Gazette 92/04**

## (54) UTILISATION DE DERIVES DE PYRIMIDINE 3-OXYDE POUR FREINER LA CHUTE DES CHEVEUX ET COMPOSITIONS TOPIQUES MISES EN OEUVRE.

(30) Priorité : **20.07.90 LU 87766**

(43) Date de publication de la demande : **12.05.93 Bulletin 93/19**

(45) Mention de la délivrance du brevet : **04.05.94 Bulletin 94/18**

(84) Etats contractants désignés : **AT BE CH DE DK ES FR GB GR IT LI NL SE**

(56) Documents cités :
**EP-A- 0 356 271**
**WO-A-89/07595**

(73) Titulaire : **L'OREAL**
**14, Rue Royale**
**F-75008 Paris (FR)**

(72) Inventeur : **HOCQUAUX, Michel**
**70, rue du Rendez-Vous**
**F-75012 Paris (FR)**
Inventeur : **DUMATS, Jacqueline**
**13, avenue A.-Croizat**
**F-93420 Villepinte (FR)**
Inventeur : **GALEY, Jean Baptiste**
**20, rue Lacépède**
**F-75005 Paris (FR)**

(74) Mandataire : **Casalonga, Axel**
**BUREAU D.A. CASALONGA - JOSSE**
**Morassistrasse 8**
**D-80469 München (DE)**

EP 0 540 629 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à l'utilisation de dérivés de pyrimidine 3-oxyde pour freiner la chute des cheveux et aux compositions contenant ces dérivés, destinées à être appliquées de façon topique sur le cuir chevelu.

On recherche depuis de nombreuses années des composés efficaces au niveau du traitement de la chute des cheveux sans présenter cependant d'effets secondaires qui pourraient être gênants lors d'une application prolongée.

On connait en particulier des compositions à base de mucopolysaccharides utilisées pour freiner la chute des cheveux.

EP-A-0 356 271 décrit des compositions contenant des dérivés de diamino-2,4-pyrimidine oxyde-3, substitués en position 6 et leur utilisation dans le traitement et la prévention de la chute des cheveux.

La demanderesse a découvert de nouveaux dérivés de pyrimidine 3-oxyde particulièrement efficaces pour freiner la chute des cheveux. Elle a constaté en particulier une augmentation du nombre de cheveux en phase anagène ou en phase de croissance et une diminution du nombre de cheveux en phase telogène ou en phase terminale du cheveu. L'augmentation du rapport de nombre de cheveux en phase anagène par rapport au nombre de cheveux en phase telogène est une indication de l'effet de ces composés sur le traitement de la chute des cheveux. Ces composés ont par ailleurs l'avantage de ne pas présenter d'effets secondaires pouvant être gênants lors d'une application prolongée.

L'invention a pour objet l'utilisation pour freiner la chute des cheveux de dérivés de pyrimidine 3-oxyde.

Un autre objet de l'invention est constitué par les compositions topiques destinées à être utilisées pour freiner la chute des cheveux.

D'autres objets de l'invention apparaîtront à la lecture de la description et des exemples qui suivent.

Les composés utilisés conformément à l'invention pour freiner la chute des cheveux répondent à la formule générale:

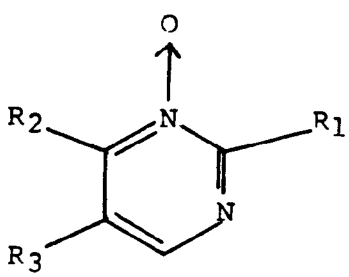

(I)

dans laquelle:

$R_1$ désigne un groupement méthyle ou $NHR_4$ dans lequel $R_4$ désigne un groupement alkyle en $C_1$-$C_4$ ou un atome d'hydrogène ;

$R_2$ désigne hydrogène ou un groupement $NHR_4$ dans lequel $R_4$ a la même signification que ci-dessus ; $R_2$ peut également désigner méthyle lorsque ; $R_1$ désigne méthyle lorsque $R_1$ désigne $NH_2$, $R_2$ ne peut pas être hydrogène;

$R_3$ peut désigner un atome d'hydrogène, un groupement alkyle en $C_1$-$C_4$ pouvant porter éventuellement:

- un groupement $OR_5$ dans lequel $R_5$ désigne un groupement alkyle en $C_1$-$C_4$,
- un noyau benzénique éventuellement substitué par un ou plusieurs groupements alcoxy en $C_1$-$C_4$ ;
- $R_3$ peut également désigner un atome d'halogène ou un groupement nitro ou amino
- et les sels d'addition d'acides physiologiquement acceptables.

Un groupement alkyle désigne de préférence, conformément à l'invention, un groupement méthyle ou éthyle, le groupement alcoxy désigne de préférence méthoxy ou éthoxy, les atomes d'halogène désignent de préférence chlore ou brome.

Les composés préférés utilisables conformément à l'invention sont les composés répondant à la formule (I) dans laquelle $R_1$ désigne amino, $R_2$ désigne amino, $R_3$ désigne hydrogène ou bien $R_1$ désigne amino, $R_2$ désigne amino et $R_3$ désigne chlore.

Les composés répondant à la formule générale (I) peuvent être obtenus par exemple, par hydrogénolyse, en présence de palladium sur charbon, de composés répondant à la formule (II) définie ci-dessous dans laquelle Z désigne un atome d'halogène et de préférence chlore ou brome.

La réduction est effectuée suivant la méthode classique décrite dans la littérature (D.J. BROWN, The pyrimidines, supplement II, Vol. 16, chapître X, page 360, Interscience Pub. 1985 ; COWDEN and WARING, Aust.

J. Chem. 34, 1539 (1981) selon le schéma réactionnel suivant :

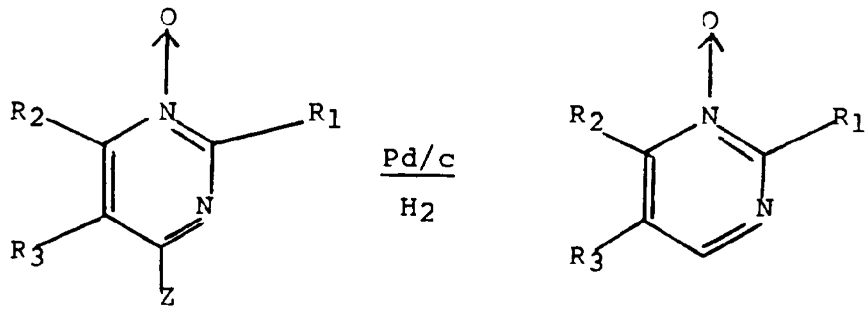

(II) (I)

Les composés de formule (I) peuvent également être utilisés sous forme de leurs sels d'addition d'acide physiologiquement acceptables tels que les sels d'acide sulfurique, chlorhydrique, phosphorique, acétique, benzoïque, salicylique, glycolique, acéturique succinique, nicotinique, tartrique, maléïque, pamoïque, méthane sulfonique, picrique, lactique, d'aminoacides et plus particulièrement d'acide acéturique.

Ces composés sont généralement utilisés pour le traitement de la chute du cheveu dans des compositions pouvant se présenter sous forme de lotion, de shampooing, de gel, de mousse, d'émulsion, de dispersion vésiculaire, de savon, de spray ou de mousse aérosol.

Les compositions destinées à une application topique sont essentiellement caractérisées par le fait qu'elles contiennent, dans un milieu physiologiquement acceptable approprié pour une application topique, au moins un composé répondant à la formule (I) ou un de ses sels d'addition d'acide définis ci-dessus.

Le composé de formule (I) est présent dans des proportions comprises entre 0,1 et 10% en poids et de préférence entre 0,2 et 5% en poids par rapport au poids total de la composition.

Le milieu physiologiquement acceptable peut être constitué par tout milieu approprié pour une application topique soit en cosmétique, soit en pharmacie qui soit compatible avec la substance active. Les composés conformes à l'invention peuvent se trouver dans ce milieu soit à l'état dissous, soit à l'état dispersés, notamment sous forme micronisée.

Le milieu physiologiquement acceptable peut être constitué par de l'eau ou un mélange d'eau et d'un solvant ou un mélange de solvants. Les solvants sont choisis parmi les solvants organiques acceptables sur le plan cosmétique ou pharmaceutique et choisis plus particulièrement parmi les alcools inférieurs en $C_1$-$C_4$ comme l'alcool éthylique, l'acool isopropylique, l'alcool tertiobutylique, les alkylèneglycols, les alkyléthers d'alkylèneglycols et de dialkylèneglycols telsque le monoéthyléther d'éthylèneglycol, le monométhyléther de propylèneglycol, le monoéthyléther de diéthylèneglycol. Les solvants, lorsqu'ils sont présents le sont dans des proportions comprises entre 1 et 80% en poids par rapport au poids total de la composition.

Le milieu peut être épaissi à l'aide d'agents épaississants habituellement utilisés en cosmétique ou en pharmacie.

Les épaississants sont présents de préférence dans des proportions comprises entre 0,1 et 5% en poids et en particulier entre 0,4 et 3% en poids par rapport au poids total de la composition.

Ces compositions peuvent également renfermer :

- des oligosaccharides estérifiés tels que ceux décrits dans EP-A-0 211 610 et EP-A-0 064 012;
- des dérivés d'acide hexosaccharique tels que ceux décrits dans EP-A-0 375 388, en particulier l'acide glucosaccharique;
- des inhibiteurs de glycosidase tels que ceux décrits dans EP-A-0 334 586, en particulier le D-glucaro 1,5-lactame;
- des inhibiteurs de glycosaminoglycanase et protéoglycanase tels que ceux cités dans EP-A-0 277 428, en particulier la L-galactono 1,4-lactone;
- des inhibiteurs de tyrosine kinase tels que ceux décrits dans EP-A-0 403 238, en particulier le 1-amido 1-cyano-(3,4-dihydroxyphényl)éthylène;
- des hyperémiants tels que :
  . les esters d'acide nicotinique dont plus particulièrement les nicotinates de benzyle et d'alkyle en $C_1$-$C_6$ et notamment le nicotinate de méthyle, de benzyle, ainsi que le nicotinate de tocophérol;
  . les bases de xanthine dont plus particulièrement la caféine et la théophylline;

. la capsicine;
- des filtres UV-A et UV-B comme les méthoxycinnamates, les dérivés de benzophénone;
- des inhibiteurs de la phosphodiestérase tels que la Visnadine® ;
- des activateurs de l'adénine cyclase tels que le Forskolin;
- des antioxydants et capteurs de radicaux libres, en particulier
  . des radicaux OH tels que le DMSO;
  . l'α-tocophérol, BHA, BHT;
  . la superoxyde dismutase (SOD);
- des agents antipelliculaires tels que l'omadine, l'octopirox;
- des agents hydratants tels que l'urée, la glycérine, l'acide lactique, les α-hydroxyacides, la thiamorpholinone et ses dérivés, des lactones;
- les agents antiséborrhéiques tels que la S-carboxyméthylcystéine, la S-benzylcystéamine et leurs dérivés, la thioxolone;
- les anti-inflammatoires stéroïdiens et non stéroïdiens tels que l'hydrocortisone, la bétaméthasone, la dexaméthasone, l'acide niflumique;
- les antiandrogènes et hormones tels que l'estriol, l'estradiol, la thyroxine, l'oxendolone, le diéthylstilbestrol;
- les rétinoïdes dont plus particulièrement l'acide t-trans rétinoïque appelé encore tretinoïne, l'isotrétinoïne, le rétinol ou vitamine A et ses dérivés, tels que l'acétate, le palmitate ou le propionate, le motretinide, l'étretinate, le t-trans rétinoate de zinc;
- les antibactériens choisis plus particulièrement parmi les macrolides, les pyranosides et les tetracyclines, et notamment l'erythromycine;
- les antagonistes de calcium dont on peut citer la Cinnarizine et le Diltiazem à titre d'exemples non limitatifs;
- des phospholipides tels que la lécithine;
- le diazoxyde (3-méthyl 7-chloro[2H]benzothiadiazine-1,2,4 dioxyde-1,1);
- les acides linoléïque et linolénique;
- l'anthraline et ses dérivés;
- l'acide 5-alkanoyl salicylique et ses dérivés tels que décrits dans le brevet FR-25 81 542;
- des activateurs de pénétration tels que le THF, le 1,4-dioxane, l'alcool oléique, la 2-pyrrolidone, le salicylate de benzyle, etc.;
- des vitamines ou provitamines tels que la β-carotène, la biotine, le panthénol et ses dérivés, la vitamine C, les vitamines $B_2$, $B_4$, $B_6$.

Ces compositions peuvent également contenir de l'AMP cyclique, le MPS.

Ces compositions peuvent également contenir des agents conservateurs, des agents stabilisants, des agents régulateurs de pH, des agents modificateurs de pression osmotique, des agents émulsifiants.

Les composés conformes à l'invention peuvent également être associés à des agents tensio-actifs dont notamment ceux choisis parmi les agents tensio-actifs non ioniques et amphotères.

Parmi les tensio-actifs non ioniques, on citera les polyhydroxypropyléthers décrits notamment dans les brevets français n° 1 477 048; 2 091 516; 2 169 787; 2 328 763; 2 574 786; les alkyl($C_8$-$C_9$)phénols oxyéthylénés comportant de 1 à 100 moles d'oxyde d'éthylène et de préférence 5 à 35 moles d'oxyde d'éthylène; les alkylpolyglycosides de formule :

$$C_nH_{2n+1}\ (C_6H_{10}O_5)_xH \qquad (III)$$

dans laquelle n varie de 8 à 15 inclus et x de 1 à 10 inclus.

Parmi les agents tensio-actifs amphotères, on citera plus particulièrement les amphocarboxyglycinates et les amphocarboxypropionates définis dans le dictionnaire CTFA, 3ème édition, 1982, et vendus, notamment, sous la dénomination MIRANOL® par la Société MIRANOL.

Les composés, selon l'invention, peuvent être introduits dans des supports qui améliorent encore l'activité au niveau de la repousse, en présentant à la fois des propriétés avantageuses sur le plan cosmétique, telles que des mélanges volatils ternaires d'alkyléther d'alkylèneglycols, en particulier d'alkyle en $C_1$-$C_4$, d'alkylène en $C_1$-$C_4$ glycol ou de dialkylèneglycol, de préférence de dialkylène en $C_1$-$C_4$ glycol, d'alcool éthylique et d'eau; les alkyléthers d'alkylène glycols préférés sont les monoéthyléthers de l'éthylène glycol, le monométhyléther du propylèneglycol, le monométhyléther du diéthylèneglycol.

On peut également utiliser des tensio-actifs cationiques et/ou anioniques.

Les composés conformes à l'invention peuvent également être introduits dans des supports gélifiés ou épaissis, tels que des supports essentiellement aqueux gélifiés par des hétérobiopolysaccharides, tels que la gomme de xanthane, les scléroglucanes ou les dérivés de cellulose, en particulier les éthers de cellulose, des supports hydroalcooliques gélifiés par des polyhydroxyéthylacrylates ou méthacrylates ou des supports essentiellement aqueux épaissis en particulier par des acides polyacryliques réticulés par un agent polyfonctionnel, tel que les Carbopol vendus par la Société GOODRICH.

L'invention a également pour objet un procédé de traitement cosmétique des cheveux ou du cuir chevelu, consistant à leur appliquer au moins une composition telle que définie ci-dessus, en vue d'améliorer l'esthétique de la chevelure.

Le traitement consiste principalement à appliquer sur les zones alopéciques du cuir chevelu d'un individu, la composition telle que définie ci-dessus.

Le mode d'application préféré consiste à appliquer 1 à 2 g de la composition sur la zone alopécique, à une fréquence de une à deux applications par jour, pendant 1 à 7 jours par semaine et ceci pendant une durée de 1 à 6 mois.

L'invention a également pour objet une composition destinée au traitement thérapeutique de la chute des cheveux, notamment de l'alopécie, cette composition correspondant à la définition des compositions à application topique définies ci-dessus.

Les exemples suivants sont destinés à illustrer l'invention.

## EXEMPLES DE COMPOSITION

### EXEMPLE 1

On prépare la composition suivante :

- 2,4-diamino pyrimidine 3-oxyde 3 g
- propylène glycol 20 g
- éthanol à 95° 30 g
- eau q.s.p 100 g

Cette composition se présente sous forme de lotion.

### EXEMPLE 2

On prépare la composition suivante :

- 2,4-diamino pyrimidine 3-oxyde 1,05 g
- nicotinate de méthyle 0,105 g
- parfum 0,26 g
- éthanol absolu 29,5 g
- eau q.s.p 100 g

Cette composition se présente sous forme de lotion.

1 à 2 g des compositions décrites aux exemples 1 et 2 sont appliqués sur les zones alopéciques du cuir chevelu, l'application étant éventuellement accompagnée d'un massage pour favoriser la pénétration, à raison d'une à deux applications par jour, pendant trois mois de traitement.

On constate une diminution de la chute des cheveux.

### EXEMPLE 3

On prépare la composition suivante :

- 2,4-diamino pyrimidine 3-oxyde 1,05 g
- parfum 0,26 g
- éthanol absolu 29,5 g
- eau q.s.p 100 g

Cette composition se présente sous forme de lotion.

### EXEMPLE 4

On prépare les lotions (A) et (B) suivantes :

#### LOTION (A)

- 2,4-diamino 5-chloropyrimidine 3-oxyde 1,5 g
- Propylèneglycol 20 g
- Ethanol 30 g

- Eau purifiée qsp 100 g

LOTION (B)

- Visnadine 3 g
- Propylèneglycol 5 g
- Ethanol absolu qsp 100 g

La lotion (A) est appliquée le matin sur les zones alopéciques du cuir chevelu en procédant si souhaité à un massage, suivi par l'application de la lotion (B) le soir. On constate au bout de quelques mois de traitement une diminution de la chute des cheveux.

EXEMPLE 5

On prépare les lotions (A) et (B) suivantes :

LOTION (A)

- Acide 5-octanoyl salicylique 0,5 g
- Ethanol 50 g
- Eau purifiée qsp 100 g

LOTION (B)

- 2,4-diamino pyrimidine 3-oxyde 2 g
- Propylèneglycol 20 g
- Ethanol 50 g
- Eau purifiée qsp 100 g

La lotion (A) est appliquée le matin sur les zones alopéciques du cuir chevelu en procédant si souhaité à un massage, suivi par l'application de la lotion (B) le soir. On constate au bout de quelques mois de traitement une diminution de la chute des cheveux.

EXEMPLE 6

On prépare les solutions (A) et (B) suivantes :

SOLUTION (A)

- 2,4-diamino pyrimidine 3-oxyde 2,5 g
- Propylèneglycol 7 g
- Ethanol absolu qsp 100 g

SOLUTION (B)

- Acide rétinoïque (tout-trans) 0,05 g
- Propylèneglycol 7g
- Ethanol absolu qsp 100 g

Les solutions (A) et (B) sont stockées dans un kit à deux compartiments.

Le mélange 50/50 des composantes (A) et (B) conduit à 1,25% de 2,4-diamino pyrimidine 3-oxyde + 0,025% d'acide rétinoïque.

On applique ce mélange sur les zones alopéciques du cuir chevelu, sans rincer la composition après application.

EXEMPLE 7

On prépare la lotion suivante :

- 2,4-diamino pyrimidine 3-oxyde 2,5 g
- Diltiazem 1 g
- Propylèneglycol 20 g

- Ethanol 55 g
- Eau purifiée qsp 100 g

Cette lotion est appliquée sur les zones alopéciques du cuir chevelu sans procéder à un rinçage subséquent.

EXEMPLE 8

On prépare la lotion suivante :

- 2,4-diamino pyrimidine 3-oxyde 2 g
- Hydrocortisone 0,5 g
- Propylèneglycol 7 g
- Ethanol absolu qsp 100 g

Cette lotion est appliquée sur les zones alopéciques du cuir chevelu sans procéder à un rinçage subséquent.

EXEMPLE 9

On prépare la composition suivante :

GEL A L'ANTHRALINE

- Anthraline 0,1 g
- Silice vendue sous la dénomination SILICA HDK N 20 par la Société WACKER CHEMIE 3 g
- Stéarate de butyle qsp 100 g

On applique cette composition sur les zones alopéciques du cuir chevelu. On laisse poser 1/2 heure le produit.

On applique le shampooing ayant la composition suivante :

- 2,4-diamino pyrimidine 3-oxyde 1 g
- Tensio-actif non-ionique préparé par condensation de 3,5 moles de glycidol sur un $\alpha$-diol en $C_{11}$ à $C_{14}$ selon FR-A-2 091 516 26 g MA
- Hydroxypropylcellulose vendue sous la dénomination KLUCEL G par la Société HERCULES 2 g
- Conservateurs qs
- Ethanol 50 g
- Eau purifiée qs 100 g

On rince le cuir chevelu ainsi traité.

EXEMPLE 10

On prépare la lotion suivante :

- 2,4-diamino pyrimidine 3-oxyde 1,5 g
- Biotine éthylester 1 g
- Propylèneglycol 20 g
- Ethanol qsp 100 g

Cette composition est appliquée sur les zones alopéciques du cuir chevelu, sans rinçage subséquent.

EXEMPLE 11

On prépare le gel niosomé suivant :

- 2,4-diamino pyrimidine 3-oxyde 0,5 g
- Huile essentielle d'Eucalyptus 1 g
- Tensio-actif non-ionique de formule : 1,9 g

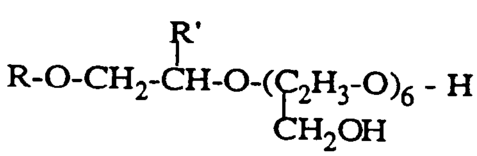

avec

$R = C_{12}H_{22}$

$R' = C_{14}H_{29}/C_{16}H_{33}$

selon FR-2 465 780.

- Na-glutamate de suif hydrogéné, vendu sous la dénomination ACYLGLUTAMATE HS 110 par la Société AJINOMOTO 0,1 g
- CARBOPOL 934 P vendu par la Société B.F. GOODRICH CORPORATION 0,3 g MA
- Conservateurs qs
- Agent de neutralisation qs pH=7
- Eau purifiée qsp 100 g

EXEMPLE 12

On prépare la lotion suivante :

- 2,4-diamino pyrimidine 3-oxyde 1,5 g
- Octopirox 0,2 g
- Ethanol 45 g
- Eau purifiée qsp 100 g

Cette composition est appliquée sur les zones alopéciques du cuir chevelu sans rinçage subséquent.

EXEMPLE 13

On prépare la composition suivante :

- 2,4-diamino pyrimidine 3-oxyde 1 g
- Filtre solaire vendu sous la dénomination UVINUL MS 40 par la Société BASF 1 g
- Vitamine F 0,1 g
- BHA 0,025 g
- BHT 0,025 g
- Tensio-actif non ionique vendu sous la dénomination POLAWAX A 31 CRODA par la Société CRODA 0,5 g
- Ethanol 50 g
- Eau purifiée qsp 100 g

Cette composition est introduite dans un récipient sous pression, à raison de 95% de composition pour 5% de propulseur hydrocarboné.

Il se forme à la sortie du récipient une mousse qui est appliquée sur les parties alopéciques du cuir chevelu.

EXEMPLE DE PREPARATION 1

2,4-diamino pyrimidine 3-oxyde (I)

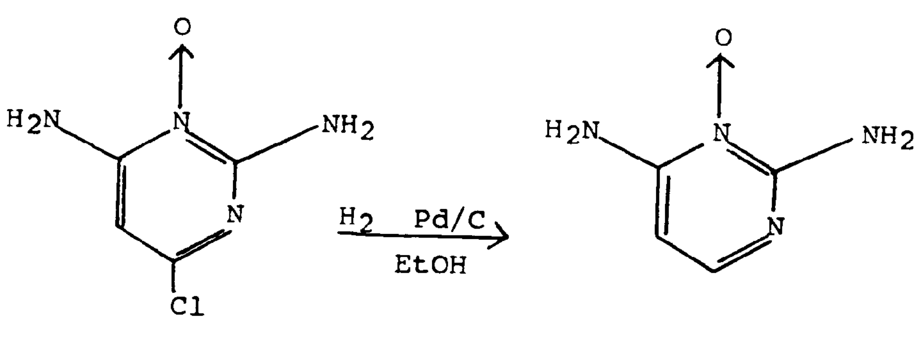


(I)

Ce dérivé est préparé par hydrogénolyse du 2,4-diamino 6-chloro pyrimidine 3-oxyde.

Mode opératoire

15 g de 2,4-diamino 6-chloro pyrimidine 3-oxyde sont dissous dans une solution contenant 5,9 g de potasse dans 1000 $cm^3$ d'éthanol absolu.

Après addition de 2,3 g de palladium sur charbon à 10%, le mélange est agité sous atmosphère d'hydrogène pendant 2 heures.

Le catalyseur est filtré, lavé avec de l'éthanol et la phase organique est évaporée.

Le résidu obtenu est recristallisé dans l'eau.

On obtient 9 g de précipité blanc (67% de rendement.

F - 192°C

Analyse élémentaire :

$C_4H_6N_4O\ H_2O$ ; PM = 144

| | C | H | N | O |
|---|---|---|---|---|
| Calculé | 33,33 | 5,56 | 38,89 | 22,22 |
| Trouvé | 33,46 | 5,60 | 39,02 | 22,50 |

Les spectres RMN $^1H$ et de masse sont conformes à la structure attendue.

EXEMPLE DE PREPARATION 2

2,4-diamino 5-chloro pyrimidine 3-oxyde

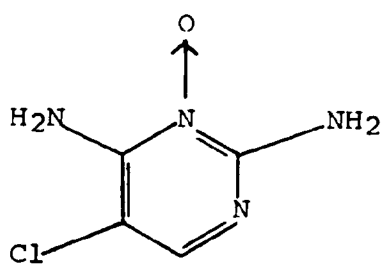

Mode opératoire

10 g de 2,4-diamino pyrimidine 3-oxyde sont dissous, dans 600 ml de méthanol, dans un tricol de 11 muni d'un réfrigérant, d'un thermomètre et d'une agitation mécanique.

On additionne, à température ambiante, 13,9 g de N-chloro succinimide. On agite 3h à 55°C.

Le milieu réactionnel est évaporé à sec puis repris dans 50 ml d'eau. Le pH est ajusté à 8 par addition de soude concentrée.

Le produit est filtré, lavé par 50 ml d'eau glacée et recristallisé dans 50 ml d'eau.

On obtient 4,5 g de 2,4-diamino 5-chloro pyrimidine 3-oxyde.

Rendement = 40% F = 220°C.

Analyse élémentaire :

$C_4H_5N_4O\ Cl$

| | C | H | N | O | Cl |
|---|---|---|---|---|---|
| Calculé | 28,93 | 3,37 | 33,75 | 12,53 | 21,40 |
| Trouvé | 28,94 | 3,46 | 33,62 | 12,47 | 21,53 |

Les spectres RMN $^1H$ et $^{13}C$ ainsi que le spectre de masse sont conformes à la structure attendue.

EXEMPLE DE PREPARATION 3

2,4-diamino 5-éthyl pyrimidine 3-oxyde

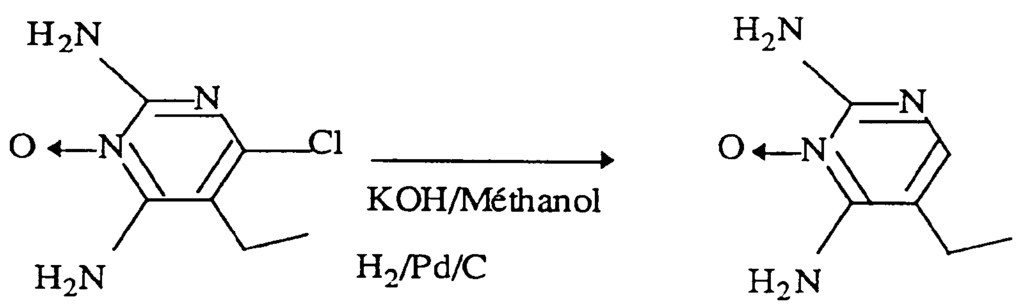


Dans une bouteille à hydrogéner de l'appareil de Parr, on place :

- 1,15 g d'hydroxyde de potassium dissous dans 300 ml de méthanol
- 0,42 g de palladium sur charbon à 10%
- 3 g de 2,4-diamino 5-éthyl 6-chloro pyrimidine 3-oxyde.

On charge la bouteille à 30 psi d'hydrogène. Après 3 heures d'agitation, on purge la bouteille par de l'azote. Le milieu réactionnel est filtré sur papier. Les filtrats sont évaporés à sec. Le résidu est recristallisé dans l'eau puis dans un mélange isopropanol/eau.

On obtient 800 mg de 2,4-diamino 5-éthyl pyrimidine 3-oxyde.
Rendement = 33%.
Analyse élémentaire : $C_6H_{10}N_4O$ ; M=154

| | C | H | N | O |
|---|---|---|---|---|
| Calculé | 46,75 | 6,49 | 36,36 | 10,39 |
| Trouvé | 46,80 | 6,55 | 36,45 | 10,47 |

Les spectres RMN $^1H$ et de masse sont conformes à la structure attendue.

EXEMPLE DE PREPARATION 4

2-méthyl 4-amino 5-chloropyrimidine 3-oxyde

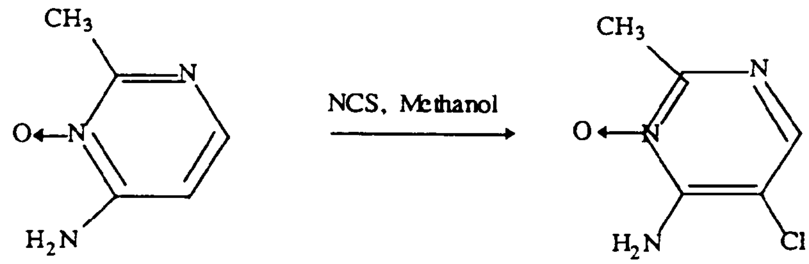


On dissout 6,3 g de 2-méthyl 4-amino pyrimidine 3-oxyde dans 150 ml de méthanol. On ajoute 7,05 g de N-chlorosuccinimide. Le milieu réactionnel est agité pendant 24 heures à température ambiante. On évapore à sec. Le résidu est repris dans 50 ml d'acétone. Le précipité est filtré puis repris dans 15 ml d'eau. On ajuste le pH à 8 par addition de soude concentrée. Après 1/2 heure d'agitation, on filtre le précipité. On recristallise dans un mélange acétonitrile/eau (70/30).

On obtient 1,20 g de 2-méthyl 4-amino 5-chloro pyrimidine 3-oxyde.

Rendement = 15%.

Point de fusion = 209°C

Analyse élémentaire : $C_5H_6Cl\ N_3O$ ; M=159,5

| | C | H | N | O | Cl |
|---|---|---|---|---|---|
| Calculé | 37,62 | 3,76 | 26,33 | 10,03 | 22,25 |
| Trouvé | 37,54 | 3,79 | 26,23 | 10,02 | 22,20 |

Les spectres RMN $^1H$ et de masse sont conformes à la structure attendue.

EXEMPLE DE PREPARATION 5

2,4-diamino 5-bromo pyrimidine 3-oxyde

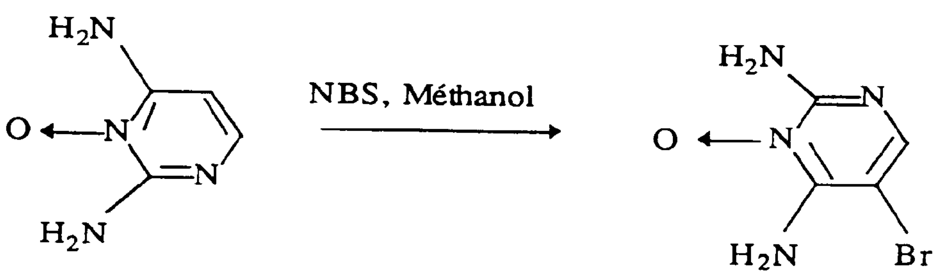


On dissout 1 g de 2,4-diamino pyrimidine 3-oxyde dans 60 ml de méthanol. On ajoute 1,55 g de N-bromosuccinimide. Le milieu réactionnel est agité pendant 2 heures à 60°C. On évapore à sec. Le résidu est repris dans 5 ml d'eau. On ajuste le pH à 8 par addition de soude concentrée. Après 1/2 heure d'agitation, le précipité est filtré sur verre fritté, rincé par 5ml d'eau puis par 2 x 25 ml d'acétone. On recristallise dans un mélange éthanol/eau (50/50).

On obtient 700 mg de 2,4-diamino 5-bromo pyrimidine 3-oxyde.

Rendement = 50%

Point de fusion: décomposition à partir de 245°C

Analyse élémentaire : $C_4H_5Br\ N_4O$ ; M=205

| | C | H | N | O | Br |
|---|---|---|---|---|---|
| Calculé | 23,41 | 2,44 | 27,32 | 7,80 | 39,02 |
| Trouvé | 23,36 | 2,45 | 27,38 | 8,01 | 38,84 |

Les spectres RMN $^1$H et de masse sont conformes à la structure attendue.

EXEMPLE DE PREPARATION 6

2-méthyl 4-amino pyrimidine 3-oxyde

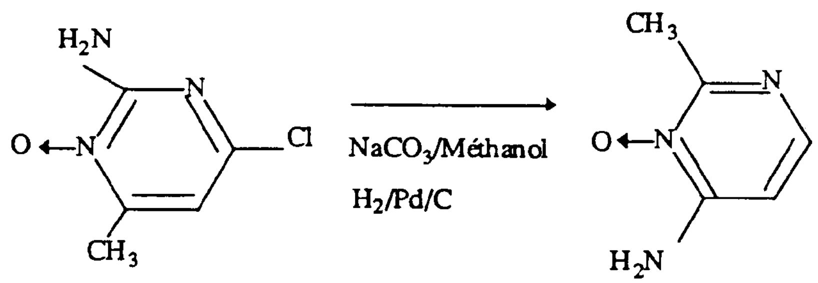


Dans une bouteille à hydrogéner de l'appareil de Parr, on place :

- 3,65 g de carbonate de sodium
- 335 ml de méthanol
- 0,8 g de palladium sur charbon à 10%
- 5 g de 2-méthyl 4-amino 6-chloro pyrimidine 3-oxyde.

On charge la bouteille à 30 psi d'hydrogène. Après 3 heures d'agitation, le système est purgé par de l'argon. On filtre le milieu réactionnel sur papier filtre. Les filtrats sont évaporés à sec. Le précipité obtenu est repris dans 50 ml d'acétonitrile bouillant. L'insoluble est filtré sur verre fritté puis recristallisé dans 60 ml d'éthanol. On recueille 1,1 g de 2-méthyl 4-amino pyrimidine 3-oxyde.

Rendement = 28%

Point de fusion = 246°C

Analyse élémentaire : $C_5H_7N_3O$ ; M=125

| | C | H | N | O |
|---|---|---|---|---|
| Calculé | 48,00 | 5,60 | 33,60 | 12,80 |
| Trouvé | 47,62 | 5,69 | 33,75 | 13,00 |

Les spectres RMN $^1$H et de masse sont conformes à la structure attendue.

EXEMPLE DE PREPARATION 7

Préparation du sel de 2,4-diamino pyrimidine 3-oxyde avec l'acide nicotinique

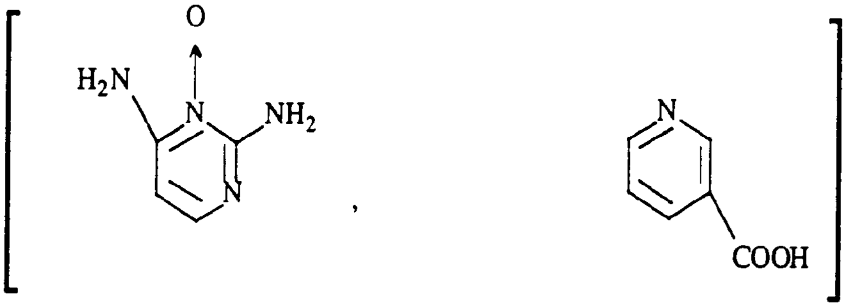

On recristallise dans 20 ml d'éthanol un mélange de 2 g de 2,4-diamino pyrimidine 3-oxyde et 1,71 g d'acide nicotinique. On récupère un solide blanc que l'on sèche pour obtenir 2,90 g de sel de2,4-diamino pyrimidine 3-oxyde et d'acide nicotinique.
Rendement = 78%
Point de fusion = 173-176°C
<u>Analyse élémentaire</u> : $C_{10}H_{11}N_5O_3$ + 0,36 $H_2O$

| | C | H | N | O |
|---|---|---|---|---|
| Calculé | 46,93 | 4,55 | 27,34 | 21,02 |
| Trouvé | 47,07 | 4,56 | 27,49 | 21,08 |

<u>EXEMPLE DE PREPARATION 8</u>

Préparation de l'acéturate de 2,4-diamino pyrimidine 3-oxyde

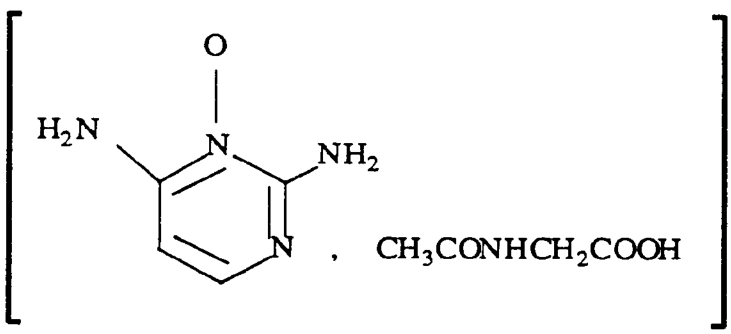
, $CH_3CONHCH_2COOH$

On recristallise dans 20 ml d'éthanol un mélange de 2 g de 2,4-diamino pyrimidine 3-oxyde et 1,62 g d'acide acéturique. On récupère un solide blanc que l'on sèche pour obtenir 3,5 g d sel d'acéturate de 2,4-diamino pyrimidine 3-oxyde.
Rendement = 96%
Point de fusion = 133-134°C
<u>Analyse élémentaire</u> : $C_8H_{13}N_5O_4$ + 0,75 $H_2O$

| | C | H | N | O |
|---|---|---|---|---|
| Calculé | 37,42 | 5,65 | 27,29 | 29,63 |
| Trouvé | 37,42 | 5,63 | 27,45 | 29,92 |

<u>EXEMPLE DE PREPARATION 9</u>

Préparation du sel de 2,4-diamino pyrimidine 3-oxyde avec l'acide 5-octanoyl salicylique

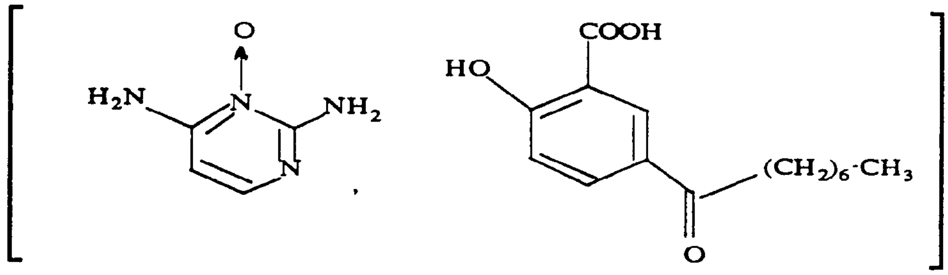

On recristallise dans 50 ml d'éthanol un mélange de 1 g de 2,4-diamino pyrimidine 3-oxyde et 1,83 g d'ac5-octanoyl salicylique. On récupère un solide blanc que l'on sèche pour obtenir 2,25 g de sel de 2,4-diamino pyrimidine 3-oxyde et d'acide 5-octanoyl salicylique.

Rendement = 79%

Point de fusion = 177°C

Analyse élémentaire : $C_{19}H_{26}N_4O_5$

| | C | H | N | O |
|---|---|---|---|---|
| Calculé | 58,45 | 6,71 | 14,35 | 20,49 |
| Trouvé | 58,50 | 6,67 | 14,27 | 20,63 |

EXEMPLE DE PREPARATION 10

Préparation de la 2,4-diamino 5-nitro pyrimidine 3-oxyde

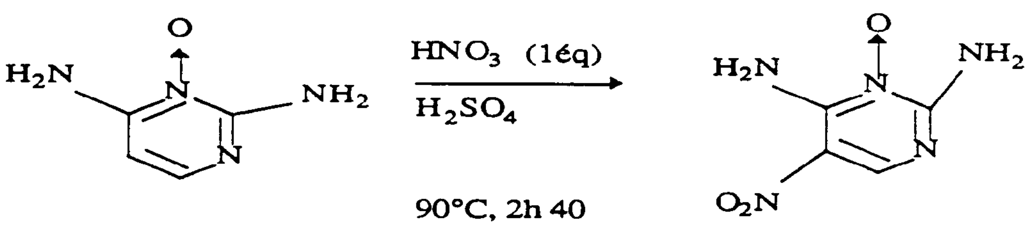


Mode opératoire

On dissout 2 g de 2,4-diamino pyrimidine 3-oxyde dans 10 ml d'acide sulfurique concentré, puis on additione goutte à goutte et à température ambiante 5 ml d'un mélange $HNO_3$ à 65% et $H_2SO_4$ concentré dans les proportions $HNO_3$ : $H_2SO_4$ = 1:4. On porte la réaction à 90°C pendant 2 heures 40 minutes.

On verse le milieu réactionnel sur 250 g de glace pilée et on neutralise par addition de 25 g de KOH en pastilles, puis avec 12 g de $NaHCO_3$ que l'on introduit par petites portions à O°C. Après filtration, le gâteau est lavé avec 3x100 ml d'eau et on le porte au reflux d'un mélange EtOH/$H_2O$ (1:1). Après filtration, on sèche le solide jaune isolé pour obtenir 1,4 g de 2,4-diamino 5-nitro pyrimidine 3-oxyde.

Rendement = 57%

Point de fusion supérieur à 300°C

Analyse élémentaire : $C_4H_5N_5O_3$ ; M=171

| | C | H | N | O |
|---|---|---|---|---|
| Calculé | 28,08 | 2,95 | 40,93 | 28,05 |
| Trouvé | 28,28 | 2,91 | 40,92 | 27,86 |

Les spectres RMN $^1H$ et de masse sont conformes à la structure attendue.

## Revendications

1. Composition destinée à une application topique caractérisée par le fait qu'elle contient dans un milieu physiologiquement acceptable approprié pour une application topique au moins un composé répondant à la formule (I) :

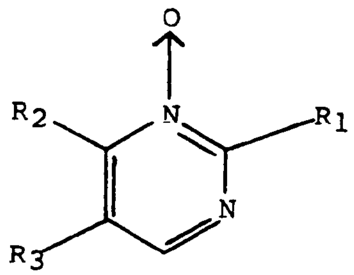

(I)

dans laquelle:

$R_1$ désigne un groupement méthyle ou $NHR_4$ dans lequel $R_4$ désigne un groupement alkyle en $C_1$-$C_4$ ou hydrogène ;

$R_2$ désigne hydrogène ou un groupement $NHR_4$ dans lequel $R_4$ a la même signification que ci-dessus ;

$R_2$ peut également désigner méthyle lorsque $R_1$ désigne méthyle ; lorsque $R_1$ désigne $NH_2$, $R_2$ ne peut pas être hydrogène;

$R_3$ peut désigner un atome d'hydrogène, un groupement alkyle en $C_1$-$C_4$ pouvant porter éventuellement:

- un groupement $OR_5$ dans lequel $R_5$ désigne un groupement alkyle en $C_1$-$C_4$,
- un noyau benzénique éventuellement substitué par un ou plusieurs groupements alcoxy en $C_1$-$C_4$ ;
- $R_3$ peut également désigner un atome d'halogène ou un groupement nitro ou amino
- et les sels d'addition d'acides physiologiquement acceptables.

2. Composition selon la revendication 1, caractérisée par le fait que le groupement alkyle désigne méthyle ou éthyle le groupement alcoxy désigne méthoxy ou éthoxy, les atomes d'halogènes désignent chlore ou brome.

3. Composition selon la revendication 1 ou 2, caractérisée par le fait que les composés de formule (I) sont choisis parmi les composés répondant à la formule (I) dans laquelle $R_1$ désigne amino, $R_2$ désigne amino, $R_3$ désigne hydrogène ou bien chlore.

4. Composition selon l'une quelconque des revendications 1 à 3, caractérisée par le fait que les sels d'addition d'acide physiologiquement acceptables sont choisis parmi les sels d'acide sulfurique, chlorhydrique, phosphorique, acétique, benzoïque, salicylique, glycolique, acéturique, succinique, nicotinique, tartrique, maléique, pamoïque, méthane sulfonique, picrique, lactique, d'aminoacides.

5. Composition selon l'une quelconque des revendications 1 à 4, caractérisée par le fait qu'elle se présente sous forme de lotion, de shampooing, de gel, de mousse, d'émulsion, de dispersion vésiculaire, de savon de spray ou de mousse aérosol.

6. Composition selon l'une quelconque des revendications 1 à 5, caractérisée par le fait que le milieu physiologiquement acceptable est un milieu dans lequel le composé de formule (I) se trouve soit à l'état dissous, soit à l'état dispersé.

7. Composition selon l'une quelconque des revendications 1 à 6, caractérisée par le fait que le milieu physiologiquement acceptable est constitué par de l'eau ou un mélange d'eau et d'un solvant choisi parmi les alcools inférieurs en $C_1$-$C_4$, les alkylènes glycols, les alkyléthers d'alkylène glycols et de dialkylène glycols présents dans des proportions comprises entre 1 et 80% en poids par rapport au poids total de la composition.

8. Composition selon l'une quelconque des revendications 1 à 7, caractérisée par le fait que le milieu est épaissi à l'aide d'agents épaississants.

9. Composition selon l'une quelconque des revendications 1 à 8, caractérisée par le fait qu'elle contient un ou plusieurs agents choisis parmi les oligosaccharides estérifiés, les dérivés d'acide hexosacchariques, les inhibiteurs de glycosidase, les inhibiteurs de glycosaminoglycanase et protéoglycanase, les inhibiteurs de tyrosine kinase, des hyperémiants, des filtres UV-A et/ou UV-B , des inhibiteurs de phosphodiestérase, des activateurs de l'adenine cyclase; des antioxydants et capteurs de radicaux libres, des agents antipelliculaires, des agents hydratants, des agents antiséborrhéiques, des agents anti-inflammatoires stéroïdiens et non stéroïdiens, des agents antiandrogènes, des hormones, des rétinoïdes, des agents antibactériens, des agents antagonistes du calcium, des phospholipides, le diazoxyde, les acides linoléique ou linolénique, l'anthraline et des dérivés anthracéniques, l'acide S-alkanoyl salicylique et ses dérivés, des activateurs de pénétration, des vitamines ou provitamines.

10. Composition selon l'une quelconque des revendications 1 à 9, caractérisée par le fait que la composition contient en plus des agents conservateurs, des agents stabilisants, des agents régulateurs de pH, des agents modificateurs de pression osmotique, des agents émulsifiants, des filtres solaires UV-A et UV-B, des agents antioxydants, des parfums.

11. Composition selon l'une quelconque des revendications 1 à 10, caractérisée par le fait qu'elle contient à titre d'agent tensio-actif des agents tensio-actifs choisis parmi les agents tensio-actifs non ioniques et amphotères.

12. Composition selon la revendication 11, caractérisée par le fait que les agents tenio-actifs non ioniques sont choisis parmi les polyhydroxypropyléthers, les alkyl($C_8$-$C_9$) phénols oxyéthylénés comportant de 1 à 100 moles d'oxyde d'éthylène, les alkylpolyglucosides de formule :

$$C_nH_{2n+1}(C_6H_{10}O_5)_xH \quad \text{(III)}$$

et que les agents tensio-actifs amphotères, sont choisis parmi les amphocarboxyglycinates et les amphocarboxypropionates.

13. Composition selon l'une quelconque des revendications 1 à 12, caractérisée par le fait que le milieu est constitué par un mélange volatil ternaire d'alkyléther d'alkylèneglycol, d'alcool éthylique et d'eau.

14. Composition selon l'une quelconque des revendications 1 à 13, caractérisée par le fait que le milieu se présente sous forme d'un gel ou d'un milieu épaissi contenant à titre d'épaississants des hétérobiopolysaccharides, des dérivés de cellulose, des polyhydroxy éthylacrylates ou méthacrylates ou des acides polyacryliques réticulés par un agent polyfonctionnel.

15. Composition selon l'une quelconque des revendications 1 à 14, caractérisée par le fait qu'elle contient également un agent tensio-actif cationique et/ou anionique.

16. Utilisation du composé répondant à la formule (I) telle que définie dans l'une quelconquedes revendications 1 à 4 dans le traitement non thérapeutique de la chute des cheveux.

17. Procédé de traitement cosmétique des cheveux ou du cuir chevelu, caractérisé par le fait que l'on applique au moins une composition telle que définie dans l'une quelconque des revendications 1 à 16 sur le cuir chevelu.

18. Composition selon l'une quelconque des revendications 1 à 16 pour son application dans le traitement thérapeutique de la chute des cheveux.

19. Utilisation de composés répondant à la formule (I) telle que définie dans l'une quelconque des revendications 1 à 4 pour la préparation d'une composition destinée à être utilisée dans le traitement thérapeutique de la chute des cheveux, en particulier de l'alopécie.

## Patentansprüche

1. Zusammensetzung zur topischen Aufbringung,
dadurch **gekennzeichnet,** daß
sie in einem physiologisch zulässigen, zur topischen Anwendung geeigneten Medium mindestens eine Verbindung der Formel (I) enthält:

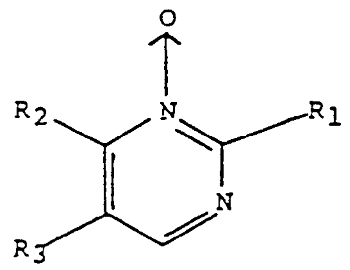

(I)

worin gilt:

$R_1$ stellt eine Methyl- oder $NHR_4$-Gruppe dar, worin $R_4$ eine $C_{1-4}$-Alkylgruppe oder ein Wasserstoffatom bedeutet;

$R_2$ stellt Wasserstoff oder eine $NHR_4$-Gruppe dar, worin $R_4$ dieselbe Bedeutung wie oben hat; $R_2$ kann auch Methyl bedeuten, wenn $R_1$ Methyl bedeutet; bedeutet $R_1$ $NH_2$, kann $R_2$ nicht Wasserstoff sein;

$R_3$ kann ein Wasserstoffatom, eine $C_{1-4}$-Alkylgruppe bedeuten, die gegenbenenfalls aufweisen kann:

- eine $OR_5$-Gruppe, worin $R_5$ eine $C_{1-4}$-Alkylgruppe bedeutet,
- einen benzolischen Kern, der gegebenenfalls mit einer oder mehreren $C_{1-4}$-Alkoxygruppen substituiert ist;
- $R_3$ kann auch ein Halogenatom oder eine Nitro- oder Aminogruppe bedeuten;
- sowie die physiologisch verträglichen Säureadditionssalze davon.

2. Zusammensetzung gemäß Anspruch 1,
dadurch **gekennzeichnet,** daß
die Alkylgruppe Methyl oder Ethyl, die Alkoxygruppe Methoxy oder Ethoxy und die Halogenatome Chlor oder Brom bedeuten.

3. Zusammensetzung gemäß Anspruch 1 oder 2,
dadurch **gekennzeichnet,** daß
die Verbindungen der Formel (I) aus Verbindungen der Formel (I) ausgewählt sind, worin $R_1$ die Aminogruppe, $R_2$ die Aminogruppe und $R_3$ Wasserstoff oder auch Chlor bedeuten.

4. Zusammensetzung gemäß jedem Anspruch 1 bis 3,
dadurch **gekennzeichnet,** daß
die physiologisch zulässigen Säureadditionssalze ausgewählt sind aus den Salzen von Schwefel-, Salz-, Phosphor-, Essig-, Benzoe-, Salicyl-, Glycol-, Acetylaminoessig-, Bernstein-, Nicotin-, Wein-, Malein-, Palmitin-, Methansulfon-, Pikrin-, Milchsäure und von Aminosäuren.

5. Zusammensetzung gemäß jedem Anspruch 1 bis 4,
dadurch **gekennzeichnet,** daß
sie in Form einer Lotion, eines Schampoos, Gels, Schaums, einer Emulsion, bläschenartigen Dispersion, Seife, eines Spray oder Aerosolschaums vorliegt.

6. Zusammensetzung gemäß jedem Anspruch 1 bis 5,
dadurch **gekennzeichnet,** daß
das physiologisch zulässige Medium ein Medium ist, worin sich die Verbindung der Formel (I) entweder in gelöstem oder dispergiertem Zustand befindet.

7. Zusammensetzung gemäß jedem Anspruch 1 bis 6,
dadurch **gekennzeichnet,** daß
das physiologisch zulässige Medium zusammengesetzt ist aus Wasser oder einer Mischung aus Wasser und einem Lösungsmittel, ausgewählt aus $C_{1-4}$-Niedrigalkoholen, Alkylenglycolen, Alkylethern von Alkylenglycolen und Dialkylenglycolen, welche in Mengenverhältnissen von 1 bis 80 Gew.% vorhanden sind, bezogen auf Gesamtgewicht der Zusammensetzung.

8. Zusammensetzung gemäß jedem Anspruch 1 bis 7,
dadurch **gekennzeichnet,** daß

das Medium mit Verdickungsmitteln verdickt ist.

**9.** Zusammensetzung gemäß jedem Anspruch 1 bis 8,
dadurch **gekennzeichnet,** daß
sie eines oder mehrere Mittel enthält, ausgewählt aus veresterten Oligosacchariden, Derivaten von Hexozuckersäuren, Inhibitoren von Glycosidase, Inhibitoren von Glycosaminoglycanase und Proteoglycanase, Inhibitoren von Tyrosinkinase, aus Blutdruckmitteln, UV-A- und/oder UV-B-Filterstoffen, Inhibitoren von Phosphodiesterase, Aktivatoren von Adenincyclase, Antioxidantien und Abfangmitteln von freien Radikalen, antifilmbildenden Mitteln, Hydratisiermitteln, antiseborrheischen Mitteln, steroiden und nicht-steroiden entzündungshemmenden Mitteln, antiandrogenen Mitteln, Hormonen, Retinoiden, antibakteriellen Mitteln, antagonistischen Mitteln des Calciums, Phospholipiden, Diazoxid, Linol- oder Linolensäuren, Anthralin und seinen anthracenischen Derivaten, S-Alkanoylsalicylsäure und seinen Derivaten, Eindringaktivatoren, Vitaminen oder Provitaminen.

**10.** Zusammensetzung gemäß jedem Anspruch 1 bis 9,
dadurch **gekennzeichnet,** daß
die Zusammensetzung ausserdem Konservierungs-, Stabilisierungs-, pH-Regulierungs-, Modifizierungsmittel des osmotischen Drucks, Emulgatoren, Sonnenfilterstoffe für UV-A und UV-B, Antioxidantien, Parfüms enthält.

**11.** Zusammensetzung gemäß jedem Anspruch 1 bis 10,
dadurch **gekennzeichnet,** daß
sie als oberflächenaktives Mittel oberflächenaktive Mittel enthält, ausgewählt aus nicht-ionischen und amphoteren oberflächenaktiven Mitteln.

**12.** Zusammensetzung gemäß Anspruch 11,
dadurch **gekennzeichnet,** daß
die nicht-ionischen oberflächenaktiven Mittel aus Polyhydroxypropylethern, oxethylierten $C_{8-9}$-Alkylphenolen, enthaltend 1 bis 100 Mol Ethylenoxid, Alkylpolyglucosiden der Formel

$$C_nH_{2n+1}(C_6H_{10}O_5)_xH \qquad (III)$$

und die amphoteren oberflächenaktiven Mittel aus Amphocarboxyglycinaten und Amphocarboxypropionaten ausgewählt sind.

**13.** Zusammensetzung gemäß jedem Anspruch 1 bis 12,
dadurch **gekennzeichnet,** daß
das Medium aus einer flüchtigen ternären Mischung aus Alkylenglycolalkylether, Ethylalkohol und Wasser zusammengesetzt ist.

**14.** Zusammensetzung gemäß jedem Anspruch 1 bis 13,
dadurch **gekennzeichnet,** daß
das Medium in Form eines Gels oder eines verdickten Mediums vorliegt, das als Verdickungsmittel Heterobiopolysaccharide, Cellulosederivate, Polyhydroxyethylacrylate oder
-methacrylate oder mit einem polyfunktionellen Mittel vernetzte Polyacrylsäuren enthält.

**15.** Zusammensetzung gemäß jedem Anspruch 1 bis 14,
dadurch **gekennzeichnet,** daß
sie auch ein kationisches und/oder anionisches oberflächenaktives Mittel enthält.

**16.** Verwendung der Verbindung der in jedem der Ansprüche 1 bis 4 definierten Formel (I) in der nichttherapeutischen Behandlung des Haarausfalls.

**17.** Verfahren zur kosmetischen Behandlung der Haare oder der behaarten Haut,
dadurch **gekennzeichnet,** daß
man mindestens eine der in jedem der Ansprüche 1 bis 16 definierten Zusammensetzung auf die behaarte Haut aufträgt.

**18.** Zusammensetzung gemäß jedem Anspruch 1 bis 16 zur Anwendung in der therapeutischen Behandlung des Haarausfalls.

19. Verwendung der Verbindungen der in jedem der Ansprüche 1 bis 4 definierten Formel (I) zur Herstellung einer Zusammensetzung zur Verwendung in der therapeutischen Behandlung des Haarausfalls, insbesondere der Alopezie.

## Claims

1. Composition intended for topical application, characterized in that it contains, in a physiologically acceptable medium appropriate for a topical application, at least one compound corresponding to the formula (I):

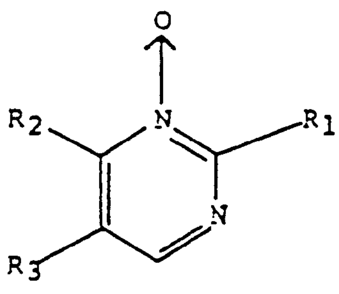

(I)

in which:

$R_1$ denotes a methyl or $NHR_4$ group in which $R_4$ denotes a $C_1$-$C_4$ alkyl group or hydrogen;

$R_2$ denotes hydrogen or a group $NHR_4$ in which $R_4$ has the same meaning as above; $R_2$ may also denote methyl when $R_1$ denotes methyl; when $R_1$ denotes $NH_2$, $R_2$ cannot be hydrogen;

$R_3$ may denote a hydrogen atom a $C_1$-$C_4$ alkyl group which may optionally carry:

- a group $OR_5$ in which $R_5$ denotes a $C_1$-$C_4$ alkyl group,
- a benzene nucleus which is optionally substituted by one or more $C_1$-$C_4$ alkoxy groups;
- $R_3$ may also denote a halogen atom or a nitro or amino group;
- and the physiologically acceptable acid addition salts.

2. Composition according to claim 1, characterized in that the alkyl group denotes methyl or ethyl, the alkoxy group denotes methoxy or ethoxy and the halogen atoms denote chlorine or bromine.

3. Composition according to claim 1 or 2, characterized in that the compounds of formula (I) are chosen from the compounds corresponding to the formula (I) in which $R_1$ denotes amino, $R_2$ denotes amino and $R_3$ denotes hydrogen or alternatively chlorine.

4. Composition according to any one of claims 1 to 3, characterized in that the physiologically acceptable acid addition salts are chosen from the salts of sulfuric, hydrochloric, phosphoric, acetic, benzoic, salicylic, glycolic, aceturic, succinic, nicotinic, tartaric, maleic, pamoic [sic], methanesulfonic, picric and lactic acid, and of amino acids.

5. Composition according to any one of claims 1 to 4, characterized in that it is provided in the form of a lotion, shampoo, gel, foam, emulsion, vesicular dispersion, soap, spray or aerosol foam.

6. Composition according to any one of claims 1 to 5, characterized in that the physiologically acceptable medium is a medium in which the compound of formula (I) is present either in the dissolved state or in the dispersed state.

7. Composition according to any one of claims 1 to 6, characterized in that the physiologically acceptable medium consists of water or a mixture of water and a solvent chosen from $C_1$-$C_4$ lower alcohols, alkylene glycols, alkylene glycol and dialkylene glycol alkyl ethers which are present in proportions of between 1 and 80% by weight relative to the total weight of the composition.

8. Composition according to any one of claims 1 to 7, characterized in that the medium is thickened by means of thickening agents.

**9.** Composition according to any one of claims 1 to 8, characterized in that it contains one or more agents chosen from esterified oligosaccharides, hexosaccharic acid derivatives, glycosidase inhibitors, glycosaminoglycanase and proteoglycanase inhibitors, tyrosine kinase inhibitors, hyperemics, UV-A- and/or UV-B-screening agents, phosphodiesterase inhibitors, adenine cyclase activators; antioxidants and free radical scavengers, antidandruff agents, moisturizing agents, antiseborrheic agents, steroidal and nonsteroidal anti-inflammatory agents, antiandrogenic agents, hormones, retinoids, antibacterial agents, calcium-antagonising agents, phospholipids, diazoxide, linoleic or linolenic acids, anthralin and anthracene derivatives, S-alkanoylsalicylic acid and its derivatives, penetration activators, and vitamins or provitamins.

**10.** Composition according to any one of claims 1 to 9, characterized in that the composition additionally contains preservatives, stabilizers, pH-regulating agents, osmotic pressure modifying agents, emulsifiers, UV-A- and UV-B-sunscreens, antioxidants and perfumes.

**11.** Composition according to any one of claims 1 to 10, characterized in that it contains, by way of surface-active agent, surface-active agents chosen from nonionic and amphoteric surface-active agents.

**12.** Composition according to claim 11, characterized in that the nonionic surface-active agents are chosen from polyhydroxypropyl ethers, oxyethylenated ($C_8$-$C_9$)-alkylphenols containing from 1 to 100 moles of ethylene oxide, alkylpolyglucosides of formula:

$$C_nH_{2n+1}(C_6H_{10}O_5)_xH \qquad \text{(III)}$$

and in that the amphoteric surface-active agents are chosen from amphocarboxyglycinates and amphocarboxy-propionates.

**13.** Composition according to any one of claims 1 to 12, characterized in that the medium consists of a ternary volatile mixture of alkylene glycol alkyl ether, ethyl alcohol and water.

**14.** Composition according to any one of claims 1 to 13, characterized in that the medium is provided in the form of a gel or a thickened medium containing, by way of thickeners, heterobiopolysaccharides, cellulose derivatives, polyhydroxyethyl acrylates or methacrylates or polyacrylic acids crosslinked by means of a polyfunctional agent.

**15.** Composition according to any one of claims 1 to 14, characterized in that it also contains a cationic and/or anionic surface-active agent.

**16.** Use of the compound corresponding to the formula (I) as defined in any one of claims 1 to 4, in the nontherapeutic treatment of hair loss.

**17.** Process for the cosmetic treatment of hair or the scalp, characterized in that at least one composition as defined in any one of claims 1 to 16 is applied to the scalp.

**18.** Composition according to any one of claims 1 to 16, for application in the therapeutic treatment of hair loss.

**19.** Use of compounds corresponding to the formula (I) as defined in any one of claims 1 to 4 for the preparation of a composition intended to be used in the therapeutic treatment of hair loss, in particular of alopecia.